# EUROPEAN PATENT SPECIFICATION

(11) **EP 4 043 059 B1**
(45) Date of publication and mention of the grant of the patent: **05.03.2025**
(21) Application number: 21211009.2
(22) Date of filing: 11.11.2020
(51) Int. Cl.: A61M 25/01, A61B 17/34, A61M 25/06, A61B 5/15, A61M 25/00, A61M 5/32, A61B 17/00, A61B 90/00

(54) **TUBULAR ELEMENT FOR MEDICAL USE**
ROHRFÖRMIGES ELEMENT ZUR MEDIZINISCHEN ANWENDUNG
ÉLÉMENT TUBULAIRE POUR UTILISATION MÉDICALE

(43) Date of publication of application: 17.08.2022
(62) Divisional of application: 20206866.4
(73) Proprietor: Fiab SpA, 50039 Vicchio Firenze (IT)
(72) Inventor: CALABRO', Alberto, 50139 FIRENZE (IT); FASANO, Antonio, 50139 FIRENZE (IT); VETTORI, Paolo, 50038 SCARPERIA E SAN PIERO (IT)
(74) Representative: Fabbriciani, Simone

(56) References cited:
- WO-A1-01/70324
- WO-A1-2017/053288
- US-A1- 2017 312 453
- US-B1- 6 796 976

## Description

This invention relates to the technical sector of devices for medical use.

In particular, the invention relates to a tubular element which can be used in surgical and out-patient operations which involve the insertion and use of probes, catheters or cannulas in a predetermined anatomical part of the body, for example blood vessels and heart cavities.

Generally speaking, the tubular elements designed to be inserted in a patient's body are delicate medical devices and designed for an equally delicate medical treatment and care purpose and therefore require particular attention in their design.

In particular, it is necessary for these elements to be made of biocompatible materials and with shapes and dimensions suitably designed to be used without risks for the patient.

It is also necessary for these devices to have structural features such as to allow the movement during use without creating risks for the health of the patient.

In this context, all the prior art solutions are subject to drawbacks and problems which sometimes render the above-mentioned tubular elements for medical use not very easy to use in certain specific situations or for particular technical reasons.

In particular, during use of such devices, it is essential to be able to check the correct positioning inside the patient's body.

For this purpose, the prior art devices have dealt in various ways with the question of visibility in fluoroscopy, but the solutions adopted have the drawback of having tips which are too rigid (steel) or which do not provide great visibility.

Exemple of prior art is given by WO 2017/053288 (A1) and by US 2017/312453 (A1), in the latter document discloses a needle shaped so as to risk damaging the cannula when it is inserted into a vein.

In this context, one of the aims is to optimise the visibility in fluoroscopy without imparting additional rigidity to the tip so as not to adversely affect the safety of use.

The perfect visibility of the tip is essential, since this is the working element of the cannula and that which must be manoeuvred with greater care so as not to adversely affect the integrity of the vessel, which, if perforated, would be a highly traumatic event which places the life of the patient at risk and which requires immediate surgical intervention.

In this context, the technical purpose could be providing a tubular element for medical use which overcomes at least some of the above-mentioned drawbacks of the prior art.

In particular, the aim would be to provide a tubular element for medical use which is able to guarantee at any time the correct and complete observation of the various parts of the element, following its insertion in the body of a patient.

The technical purpose indicated and the aims specified are substantially achieved by a tubular element for medial use comprising the technical features described in one or more of the appended claims.

The invention describes a tubular element for medical use having a main body and a pointed end and optionaly comprising a marker applied at least at an angular portion of the wall of the tubular element which extends from the pointed end to at least a portion of the main body.

Advantageously, the tubular element described may comprise a significantly radio-opaque marker which is not applied solely on its distal end, but also on at least part of the main body, allowing it to be viewed once inserted inside the body of the patient.

The invention also relates to a percutaneous extrusion cannula configured for the complete or partial extraction of medical devices, both subcutaneous (for example of the neurological type) and from a cardiac chamber.

In particular, the cannula comprises a tubular element according to the invention as defined in claim 1.

The dependent claims, relate to different embodiments of the invention.

Further features and advantages of this invention are more apparent in the detailed description below, with reference to a preferred, non-restricting, embodiment of a tubular element for medical use as illustrated in the accompanying drawings, in which:

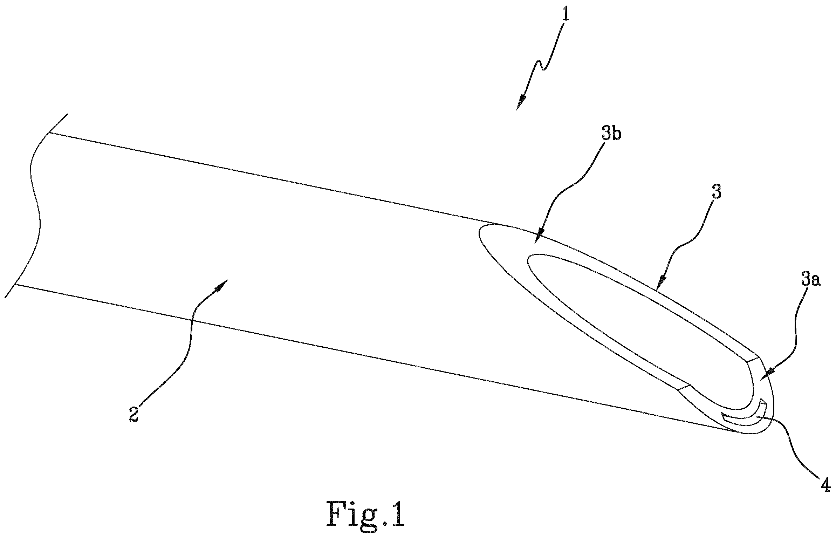

- Figure 1 is a perspective view of the end of the tubular element according to the invention designed to be inserted in the body of a patient;

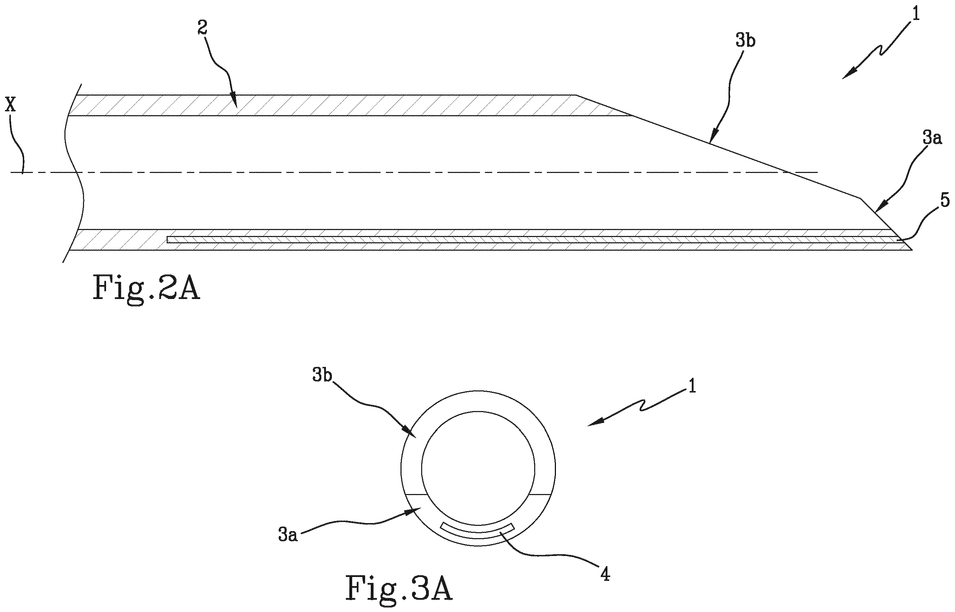

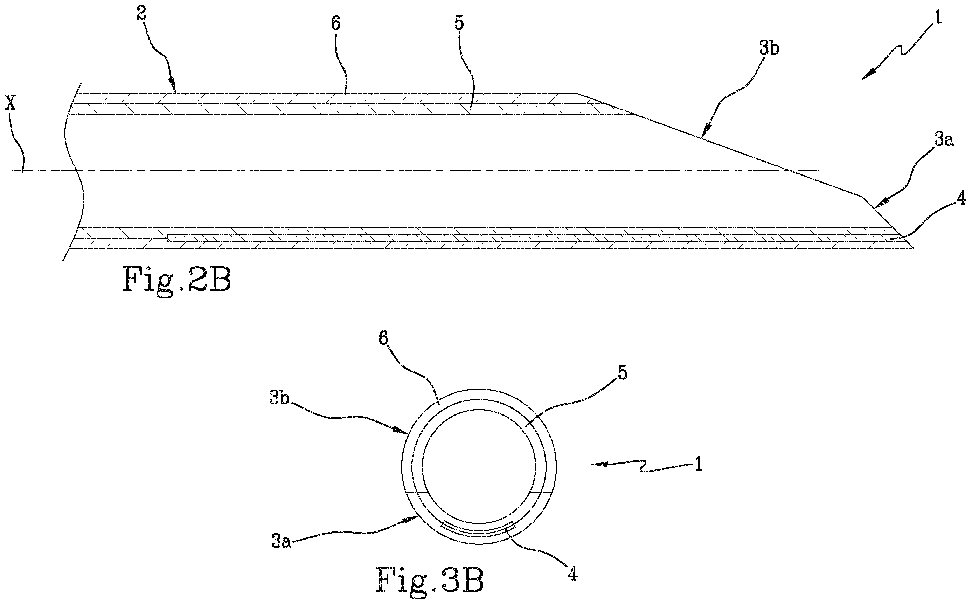

- Figures 2A-B are axial cross-sections of the end of Figure 1, according to two respective embodiments;
- Figures 3A-B show a radial cross-section of the tubular element according to the embodiments, respectively, of Figures 2A-B.

In the accompanying drawings, the numeral 1 denotes in general a tubular element for medical use according to the invention, hereinafter referred to as element 1.

Hereinafter, by way of a non-limiting example, the term tubular element for medical use will be used to refer, for example, to probes, catheters and cannulas which can be used during medical operations.

In particular, the element 1 comprises a main body 2, a pointed end 3 and a marker 4.

The main body 2 has a main axis of extension "X" and defines a gripping portion, preferably at a proximal end of the element 1, by which a user can handle the element 1, in particular for inserting it into or extracting it from the body of a patient.

The pointed end 3 is in a distal position and is configured to allow and promote insertion and movement of the subcutaneous element 1, preferably inside a cardiovascular conduit.

For this reason, for the purpose of the invention, the distal and proximal terms are used with reference to a configuration of use of the element wherein the pointed end 3 is the end designed to be inserted into the body of the patient, whilst the proximal end is the end designed to remain outside the body of the patient and preferably allow the retaining and the manipulating of the element 1 by the user.

The element 1 also comprises a marker 4, that is to say, a substance different from the materials which make the rest of the element 1 and which can be detected by suitable detection systems.

In particular, the marker may comprise a radio-opaque agent.

If necessary, the marker may further or alternatively comprise at least one between: colouring agent, opaque agent, magnetising agent, fluorescent agent, luminescent agent etc.

The marker 4 is applied at least at an angular portion of the side wall of the tubular element 1 which extends from the pointed end 3 to at least a portion of the main body 2.

According to an embodiment, the marker 4 is in the form of a continuous strip of radio-opaque material which occupies only an angular portion of the side wall of the element 1 which extends from the pointed end 3 to at least a portion of the main body 2.

In general, the marker 4 is applied in such a way as to extend at least partly along the main body 2 as well as on the part of the element 1 which defines the pointed end 3.

Preferably, the marker 4 is applied at a corner portion of the side wall of the element 1 which extends on the entire main body 2.

In other words, the entire element 1, therefore the entire main body 2 and the pointed end 3, has the marker 4 applied along its entire length, in such a way as to make its entire profile detectable, irrespective of the length for which it is inserted inside the body of the patient.

Preferably, the marker 4 is at least partly incorporated in the side wall, that is to say, the marker 4 is applied inside the side wall of the element 1.

According to a possible embodiment, not illustrated in the accompanying drawings, the element 1 comprises a first marker 4 applied at the pointed end 3 and a second marker 4 at the applied at the main body 2.

These markers 5 may have different detecting characteristics, that is to say, they are susceptible to generate different measuring signals which allow them to be distinguished in a clear manner.

In this way it is possible to detect different specific signals for the various portions which make up the element 1, thus obtaining precise information which makes it possible to define in a clear and immediate manner the actual position of the element 1 inside the body of the patient, in particular of the pointed end 3 and of the main body 2 separately.

Advantageously, the invention achieves the preset aims overcoming the drawbacks of the prior art by providing the user with a tubular element 1 for medical use wherein the presence of the marker applied not only on the tip of the element 1, but at least partly also on its main body 2 makes it possible to obtain, during use of the element 1, an overall and immediate view of the arrangement of the various elements introduced inside the patient's body. In particular, the high radio-opaqueness of the marker makes the tip clearly visibility in fluoroscopy, which must be manoeuvred very carefully, despite its reduced surface area compared with that of the main body.

According to the invention, the pointed end 3 comprises a distal end 3a and an intermediate portion 3b.

The distal end 3a has a front edge inclined relative to the main axis of extension "X", in such a way as to form with it a first operating angle α.

In other words, the operating angle defines the angular amplitude adopted by the element 1 at the distal end 3a.

In an example, the first operating angle α is between 40° and 50°, and 45° as defined in the present invention. 45°.

Consequently, the distal end 3a has a pointed shape, that is to say, it defines a tip with which it is possible to effectively engage the calcified tissue concretions which immobilise the devices to be explanted.

This function is particularly important if the element 1 is used to extract electro-catheters/catheters implanted in a heart cavity. In fact, the tip allows detachment of the sheath (constituting the outer covering of the electro-catheters) and/or the metallic parts (electrodes) from the tissues in which they are inserted and in which they could have been positioned following adherence and/or calcification processes, operating in fact like a chisel.

Preferably, the distal end 3a has an axial length of between 0.5 mm and 5 mm.

The intermediate portion 3b performs, on the other hand, a function of connecting between the distal end 3a and the main body 2.

Moreover, the intermediate portion 3b also has a front edge inclined relative to the main axis of extension "X", in such a way as to form a second operating angle β, which is different to the first operating angle α. In an example, the second operating angle β is between 10° and 30°, and 20° as defined in the present invention.

Preferably, the intermediate portion 3b has an axial length of between 1.5 mm and 25 mm.

In other words, the part of the element designed to promote the insertion into the body of a patient has two different angles α, β.

According to this embodiment, it is also possible to apply markers 4 of different types on the distal end 3a and on the intermediate portion 3b, in such a way that they can be detected separately.

Advantageously, synergic effect provided by the simultaneous presence of two different operating angles α, β makes it possible to better use the advantages provided by both without the relative drawbacks.

In particular, the first operating angle α, which is preferably greater in size, provides the distal end 3 with sufficient strength to allow the element 1 to operate correctly without being damaged, whilst the second operating angle β, which is preferably smaller in size, guarantees ease of insertion, improving manoeuvrability.

The element 1 may also comprise an inner tubular layer 5 and an outer tubular layer 6.

The inner tubular layer 5 defines an inner lateral surface of the element 1.

The outer tubular layer 6 is fitted around the inner tubular layer 5 and defines an outer side surface of the element 1.

In other words, the element 1 has a segmented side wall, that is, made by radially superposing several tubular layers 5, 6.

According to a preferred example, the inner tubular layer 5 and the outer tubular layer 6 are made using different materials.

In particular, the materials which can be used can be biocompatible plastic materials suitably selected in particular according to their elastic properties (flexibility and twisting).

By way of a non-limiting example, the tubular layers 5, 6 may be made of Polycarbonate (PC), Polypropylene (PP), Polyethylene (PET), Polyacetal resin (POM), Polyamide (PA), Polyether ether ketone (PEEK), Polytetrafluoroethylene (PTFE).

It is therefore possible to make the element 1 using materials which have different elastic characteristics, flexibility/twisting in particular, in such a way as to modulate in a precise and accurate manner the mechanical properties in particular, as a function of the particular operating context in which the element 1 must be used.

Moreover, the inner tubular layer 5 has a different thickness to a radial thickness of the outer tubular layer 6.

In this way, the difference in thickness also allows the overall rigidity of the element 1 to be modulated.

In particular, the inner tubular layer 5 has a radial thickness of between 0.10 mm and 0.25 mm, whilst the outer tubular layer 6 has a radial thickness of between 0.10 mm and 0.25 mm. It should be noted, therefore, that the element may have tubular layers 5, 6 made of different materials and/or with different radial thicknesses.

In particular, the inner tubular layer 5 and/or the outer tubular layer 6 have a radial thickness which may vary and be suitably modulated along the main axis of extension "X" of the tubular element 1.

In other words, it is possible that the element 1 has axial portions wherein the inner tubular layer 5 and the outer tubular layer 6 have radial thicknesses which vary, giving the element overall mechanical properties which vary as a function of the radial thicknesses.

Similarly, the material with which the inner tubular layer 5 and/or the outer tubular layer 6 are made may also be modified along at least some portions of the total length of the element 1.

The element 1 may also comprise at least one intermediate tubular layer, not illustrated in the accompanying drawings, interposed between the inner tubular layer 5 and the outer tubular layer 6.

In other words, it is possible to further improve the mechanical properties of the element 1 introducing further tubular layers, the radial thickness and material of which can be suitably selected so as to modify the overall rigidity of the element 1.

It is also possible to make different portions of the element 1 using different combinations of materials and/or thicknesses for the various tubular layers 5, 6 which make it up, in such a way as to locally modulate the rigidity and/or other mechanical properties of the element.

In this context, the marker 4 is preferably interposed between the inner tubular layer 5 and the outer tubular layer 6.

In this way, the marker 4 is completely enclosed between the inner tubular layer 5 and the outer tubular layer 6, being incorporated inside the side wall of the element.

This aspect advantageously facilitates the process for making the element, since it is possible to make both the two tubular layers 5, 6 and the marker 4 simultaneously and prepare the inner tubular layer 5, apply the marker 4 and then make the outer tubular layer 6 to cover both.

Advantageously, the presence of several concentric tubular layers made of different materials and/or thicknesses allows the mechanical properties of the element to be modified in a precise and accurate manner, thus allowing elements 1 to be made specially designed to operate in specific situations.

For example, one of these concentric layers (typically the outermost one and/or the innermost one) may be identified in a suitable coating (hydrophilic, fluoropolymeric, etc.) with biomedical grade materials designed to minimise the possible friction which the element 1 may encounter inside the blood vessel, that is, of another similar cannula.

The invention also relates to a percutaneous extrusion cannula configured for the complete or partial extraction of medical devices, both subcutaneous (for example of the neurological type) and from a cardiac chamber.

In particular, the cannula comprises a tubular element as described, that is to say, which incorporates one or more of the technical features indicated above.

## Claims

1. A tubular element (1) for the insertion of a probe, a catheter or a cannula inside a cardiovascular conduit, comprising a pointed end (3) consisting of:
- a distal end (3a) having a front edge inclined relative to the main axis of extension (X) in such a way as to form a first operating angle (α);
- an intermediate portion (3b) configured to join the distal end (3a) to the main body (2) and having a front edge inclined with respect to the main axis of extension (X) in such a way as to form a second operating angle (β) which is different from said first operating angle (α);
the first operating angle (α) is between 40° and 50°, and
the second operating angle (β) is between 10° and 30°;
**characterized in that**
said first operating angle (α) is equal to 45° and said second operating angle (β) is equal to 20°.

2. The element (1) according to claim 1, wherein said distal end (3a) has an axial length of between 0.5 mm and 5 mm and wherein the intermediate portion (3b) has an axial length of between 2.5 mm and 25 mm.

3. The element (1) according to claim 1 or 2, wherein said pointed end (3) is arranged to be the extreme limit, on the one hand, of a marker (4) applied at least at an angular portion of the side wall of the tubular element (1);
said marker (4) extending from said pointed end (3) to at least a portion of the main body (2) of the tubolar element (1).

4. The element according to any of claims 1-3, wherein the marker (4) is applied at an angular portion of the side wall of the tubular element of the entire main body (2).

5. A percutaneous extraction cannula configured for the total or partial extraction of medical devices implanted below the skin or in organic cavities, for example in heart chambers, comprising a tubular element (1) according to any one of the preceding claims 1-4.

## Patentansprüche

1. Rohrförmiges Element (1) zum Einführen einer Sonde, eines Katheters oder einer Kanüle in eine Herz-Kreislauf-Leitung, umfassend ein spitzes Ende (3), bestehend aus:
- einem distalen Ende (3a) mit einer Vorderkante, die relativ zur Haupterstreckungsachse (X) derart geneigt ist, dass sie einen ersten Arbeitswinkel (α) bildet;
- einem Zwischenabschnitt (3b), der ausgelegt ist, um das distale Ende (3a) mit dem Hauptkörper (2) zu verbinden, und eine Vorderkante aufweist, die in Bezug auf die Haupterstreckungsachse (X) derart geneigt ist, dass sie einen zweiten Arbeitswinkel (β) bildet, der sich von dem ersten Arbeitswinkel (α) unterscheidet;
der erste Arbeitswinkel (α) zwischen 40° und 50° beträgt, und
der zweite Arbeitswinkel (β) zwischen 10° und 30° beträgt;
**dadurch gekennzeichnet, dass**
der erste Arbeitswinkel (α) gleich 45° und der zweite Arbeitswinkel (β) gleich 20° ist.

2. Element (1) nach Anspruch 1, wobei das distale Ende (3a) eine axiale Länge zwischen 0,5 mm und 5 mm aufweist und wobei der Zwischenabschnitt (3b) eine axiale Länge zwischen 2,5 mm und 25 mm aufweist.

3. Element (1) nach Anspruch 1 oder 2, wobei das spitze Ende (3) so angeordnet ist, dass es einerseits die äußerste Grenze einer Markierung (4) ist, die mindestens an einem Winkelabschnitt der Seitenwand des rohrförmigen Elements (1) angewendet wird;
wobei sich die Markierung (4) von dem spitzen Ende (3) zu mindestens einem Abschnitt des Hauptkörpers (2) des rohrförmigen Elements (1) erstreckt.

4. Element nach einem der Ansprüche 1-3, wobei die Markierung (4) an einem Winkelabschnitt der Seitenwand des rohrförmigen Elements des gesamten Hauptkörpers (2) angewandt ist.

5. Perkutane Extraktionskanüle, die für die vollständige oder teilweise Extraktion von medizinischen Vorrichtungen ausgelegt ist, die unter der Haut oder in organischen Hohlräumen, beispielsweise in Herzkammern, implantiert sind, umfassend ein rohrförmiges Element (1) nach einem der vorhergehenden Ansprüche 1-4.

## Revendications

1. Élément tubulaire (1) pour l'insertion d'une sonde, d'un cathéter ou d'une canule à l'intérieur d'un conduit cardiovasculaire, comprenant une extrémité pointue (3) constituée de :
- une extrémité distale (3a) présentant un bord avant incliné par rapport à l'axe principal d'extension (X) de manière à former un premier angle fonctionnel (α) ;
- une partie intermédiaire (3b) configurée pour relier l'extrémité distale (3a) au corps principal (2) et présentant un bord avant incliné par rapport à l'axe principal d'extension (X) de manière à former un second angle fonctionnel (β) différent dudit premier angle fonctionnel (α) ;
le premier angle fonctionnel (α) est compris entre 40 et 50°, et
le second angle fonctionnel (β) est compris entre 10 et 30° ;
**caractérisé en ce que**
ledit premier angle fonctionnel (α) est égal à 45° et ledit second angle fonctionnel (β) est égal à 20°.

2. Élément (1) selon la revendication 1, dans lequel ladite extrémité distale (3a) a une longueur axiale comprise entre 0,5 et 5 mm et dans lequel la partie intermédiaire (3b) a une longueur axiale comprise entre 2,5 et 25 mm.

3. Élément (1) selon la revendication 1 ou 2, dans lequel ladite extrémité pointue (3) est disposée pour être la limite extrême, d'une part, d'un marqueur (4) appliqué au moins à une partie angulaire de la paroi latérale de l'élément tubulaire (1) ;
ledit marqueur (4) s'étendant de ladite extrémité pointue (3) à au moins une partie du corps principal (2) de l'élément tubulaire (1).

4. Élément selon l'une quelconque des revendications 1-3, dans lequel le marqueur (4) est appliqué à une partie angulaire de la paroi latérale de l'élément tubulaire de tout le corps principal (2).

5. Canule d'extraction percutanée configurée pour l'extraction totale ou partielle de dispositifs médicaux implantés sous la peau ou dans des cavités organiques, par exemple dans des chambres cardiaques, comprenant un élément tubulaire (1) selon l'une quelconque des revendications précédentes 1-4.
